# EUROPEAN PATENT SPECIFICATION

(11) **EP 1 703 879 B1**
(45) Date of publication and mention of the grant of the patent: **08.04.2009**
(21) Application number: 04789529.7
(22) Date of filing: 29.09.2004
(51) Int. Cl.: A61F 13/15

(54) **CUSTOMIZABLE ABSORBENT ARTICLE WITH EXTENSIBLE LAYERS**
ANPASSUNGSFÄHIGER ABSORBIERENDER ARTIKEL MIT DEHNBAREN SCHICHTEN
ARTICLE ABSORBANT ADAPTABLE A COUCHES EXTENSIBLES

(30) Priority: 30.12.2003 US 750404
(43) Date of publication of application: 27.09.2006
(73) Proprietor: KIMBERLY-CLARK WORLDWIDE, INC., Neenah, WI 54956 (US)
(72) Inventor: PRICE, Cindy, L., Appleton, Wisconsin 54913 (US); DIPALMA, Joseph, Neenah, Wisconsin 54956 (US); WALLAJAPET, Palani, Raj, Neenah, Wisconsin 54956 (US); MOCADLO, Cheryl, New London, Wisconsin 54961 (US); WEINHEIMER, Amy, Hillsboro, OR 97124 (US)
(74) Representative: Davies, Christopher Robert
(86) International application number: PCT/US2004/032939
(87) International publication number: WO 2005/067845

(56) References cited:
- EP-A- 1 260 205
- WO-A-00/19956
- WO-A-97/01996
- WO-A-97/39711
- GB-A- 2 383 957
- US-A- 6 042 575
- US-A1- 2003 023 213

## Description

### BACKGROUND

The present invention relates generally to absorbent articles for absorbing and retaining human exudates, and in particular, to an absorbent article that is extensible, allowing the user to customize the length, width, and or thickness of the article with substantially sustained deformation of the garment facing layer, the body facing layer, and/or the absorbent core. Prior art absorbent articles are disclosed in WO 97/01996, WO 97/39711 and US 6,042,575.

Absorbent articles such as sanitary napkins, incontinent garments, incontinent shields, and the like are designed to be worn as part of an absorbent garment or independently in a user's undergarment and adjacent a user's body to absorb body fluids such as menses, blood, urine, and other excrements. Conventional absorbent articles have predetermined thicknesses or shapes, However, many conventional absorbent articles do not function well with the variety of users' bodies or needs. Thus, a consumer must purchase a variety of different articles for the various needs throughout, for example, an individual's menstrual cycle. An article that may be suitable for day time wear may not be suitable for night time wear due to the changes in body positioning, direction of fluid flow and the like. Therefore, it would be desirable to have an absorbent article that could be adjusted or customized for the individual user's needs,

In addition, conventional incontinent shields and sanitary napkins may not function well with the variety of user's undergarments. For example, conventional articles may not stretch out with the user's undergarments as they are being pulled up into place or during body movement. An absorbent article that could be stretched to a point of sustained deformation by the consumer can therefore be customized to fit a particular consumer's underwear and body. Accordingly, conventional articles may not provide the desired levels of fit, absorbency and comfort. The present invention is an absorbent article that will remedy these, and other, problems of the prior endeavors, These attributes will become clear as the present invention is more thoroughly discussed in this application.

### SUMMARY

Briefly stated, in one aspect, the invention includes a customizable absorbent article having an extensible body facing layer and an extensible garment facing layer, both having a first end and a second end. The first and second ends are spaced along a longitudinal axis and the body facing and garment facing layers are extensible. Preferably, the body facing and garment facing layers are extensible along the longitudinal axis, but may also be extensible along a literal axis or in a Z direction. The article further includes a non-extenslble absorbent core which is disposed between the body facing layer and the garment facing layer. The absorbent core is affixed to at least one of the body facing and the garment facing layer at at least one location between the first and second ends.

### BRIEF DESCRIPTION OF THE DRAWINGS

FIG. 1 is a top perspective view of one aspect of the present invention;
FIG. 2 is a cross sectional view of one aspect of an article of the present invention;
FIG. 3 is a cross sectional view of another aspect of an article of the present invention;
FIGS. 4A and 4B are schematic representations of an absorbent core of the present invention in pre-use and in-use conditions, respectively;
FIG. 5 is a bottom view of one aspect of the present invention; and
FIG. 6 is a bottom view of another aspect of the present invention.

### DETAILED DESCRIPTION OF THE INVENTION

As used herein, the term "airlaid" refers to the process of producing an absorbent material where unlike components are conveyed in an air-stream and homogenously mixed or provided in a stratified configuration and then bonded together. For example, this may include, but is not limited to, the mixture of pulp fibers, synthetic fibers, superabsorbent materials and binder material. The binder material is often, but not limited to, synthetic bicomponent binder fibers and or latexes. There are a number of commercial processes available to product airlaid absorbent structures. For example, airlaid processes are available from Danweb Corp. having offices in Risskov, Denmark, and from M&J Forming Technologies having offices in Horsens, Denmark. Examples of suitable products and the process for forming them are described in U.S. Pat No. 4,640,810 to Laursen et. al., U.S. Pat. No. 4,494,278 to Kroyer et. al., U.S. Pat. No. 4,351,793 to Day, and U.S. Pat. No. 4,264,289 to Da .

An airlaid process provides a mixture of raw materials and the ability to add synthetic fibers and/or binder agents to the mixture to stabilize the resultant absorbent. As a stabilizer, binders reduce the amount of wet collapse in the structure and maintain a lower density in the saturated state. That is, the binder assists the absorbent matrix in maintaining its integrity even under load or while saturated. In addition, the resulting structure has both a higher dry and wet tensile strength than a corresponding structure without a binding agent.

The term "body-facing" should not be interpreted to mean in contact with the body of the user, but rather simply means the side that would face toward the body of the user, regardless of whether an undergarment is actually being worn by the user and regardless of whether there are or may be intervening layers between the component and the body of the user. Likewise, the term "garment-facing" should not be interpreted to mean in contact with the garments of the user, but rather simply means the side that faces away from the body of the user, and therefore toward any outer garments that may be worn by the user, regardless of whether the undergarment is actually being worn by a user, regardless of whether any such outer garments are actually worn and regardless of whether there may be intervening layers between the component and any outer garment.

As used herein, the "crotch region" of an absorbent article refers to the generally central region that will be in contact with the crotch of a user, year the lowermost part of the torso, and resides between the front and rear portions of the article. Typically, the crotch region generally extend in the longitudinal direction depending on the function of the absorbent article.

As used herein, the term "customizable" refers to an article or material that the user can alter, after purchase to better suit their needs. For example, if a user wishes to increase the length of an absorbent article for use at night, the article may be customized to meet this wish. If the user desires more absorbent material in one area or another of the absorbent article, the user may adjust the thickness or bulk of the absorbent material to meet this need. An article may be customized through manual deformation by the user or through the natural range of motion experienced by the material during use of the article.

As used here, the term "extensible" refers to a material that is capable of elongation when subjected to an applied tensile force. The material also is preferably capable of providing a selected, sustained deformation when subjected to an applied tensile force and-then allowed to relax after removal of the tensile force. Extensible materials are preferably capable of retracting minimally or not at all, due in part to the condition of hysteresis, along the X, Y, or Z axis of the absorbent article, when subjected to an applied tensile force and then allowed to relax after removal of the tensile force. Preferably the sustained deformation is substantially permanent deformation. The selected elongation and sustained deformation preferably occur at least along the longitudinal cross-direction of the material, although it should be understood that it also could occur along the lateral direction, the Z direction or all three directions.

It should be understood that the term "longitudinal," as used herein, means of or relating to the length or the lengthwise direction. The term "laterally," as used herein, means situated on, directed toward or running from side to side in a direction substantially perpendicular to the lengthwise direction. The term "lateral" or "laterally", as used herein, may also include movement in the X-Y plane.

The term "pledget" refers to an absorbent layer that has a length, width and/or thickness that is less than the length, width, and/or thickness of at least one of the layers to which it is adjacent.

The phrase "pre-use" refers to the state of the absorbent article before the user has customized the article to fit his or her individual needs. The phrase "in-use" refers to the state of the absorbent article after the user has adjusted the dimensions of the absorbent article to his or her desired configuration.

The phrase "releasable" refers to the characteristic of one or more elements being, securely but not permanently, affixed to one another. The required separation force is typically beyond that encountered through normal wear of an absorbent article in the undergarment of the user.

The-term "superabsorbent" refers to a water-swellable, water-insoluble, organic or inorganic material capable, under the most favorable conditions, of absorbing at least about 15 times its weight and, more desirably, at least about 30 times its weight of an aqueous solution containing 0.9 weight percent sodium chloride. The super absorbent materials can be natural, synthetic, and modified natural polymers and materials. In addition, the super absorbent materials can be inorganic materials, such as silica gels, or organic compounds such as crosslinked polymers. Examples of superabsorbents are disclosed in U.S. Patent No. 5,609,588 to DiPalma et. al., column 8; line 1 to line 57, which portion is incorporated herein by reference. A further example of a superabsorbent material is FAVOR SXM-880 available from Stockhausen Inc. 2401 Doyle Street, Greensboro, NC 27406, USA.

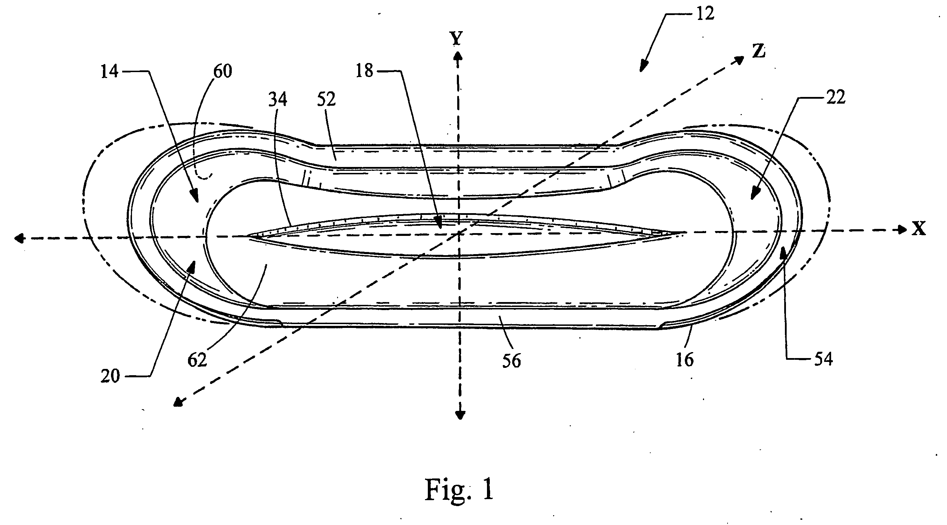

Referring now to FIG. 1, the absorbent article 12 of the present invention is customizable to the users needs. The article 12 includes a body facing layer 14, a garment facing layer 16 (See FIGS. 9 and 10) and an absorbent core 18. The body facing layer 14 is designed to contact the body of the user and desirably is liquid-permeable. The garment facing layer 16 is generally liquid-impermeable and is designed to face the inner surface, i.e. the crotch region, of the user's underwear (not shown). The body facing layer 14 and the garment facing layer 16 have first and second ends 20 and 22 and are desirably extensible along a longitudinal axis, X, but may also be extensible along a lateral axis, Y. As shown in FIG. 1, the body facing 14 and the garment facing layers 16 are manually movable from a pre-use condition to an in-use condition, The user may extend the body facing 14 and garment facing layers 16 along the longitudinal axis X to adjust the length of the article 12 to a desired configuration. The amount of applied pressure needed to extend the layers is only that sufficient to move the layers 14 and 16 into the in-use condition. For example, the body facing layer 14 and the garment facing layer 16 are extensible in that they are capable of providing an elongation of at least about 1 cm when subjected to a tensile force of 11.8 grams per centimeter (g/cm), and further provide a substantially permanent deformation of at least about 20% when subjected to a tensile force of 19.70 g/cm and is then allowed to relax under a zero applied stress for a period of 1 minute. The length (cm) is measured along the test sample, perpendicular to the applied tensile force,

Various extensible body facing materials include necked or creped spunbond and sheath/core polypropylene/KRATON elastomeric bicomponent spunbond, described in U.S. 2004/01/0442 A1 to Rhim et al. and U.S. Patent No. 6,150,002 to Eugenio Go Verona. Various extensible garment facing materials are described in U.S. Patent No. 6,096,014 to Haffner et al. (extensible biaxial film). Other suitable materials for use as the body facing and garment facing layers may include materials composed (entirely or partially) of elastomeric polymers that impart extensibility to the web, or in the case of nonextensible materials, such as nonwoven webs, laminates, spunbond, meltblown or bonded-carded web composed of synthetic polymer filaments or fibers, such as polypropylene, polyethylene, polyesters, or the like, perforated films, webs of natural polymer filaments or fibers such as rayon or cotton, be constructed by such a means as to allow for extensibility of the layer.

The body facing layer 14 may include at least one aperture 34 disposed in approximately the center of the body facing layer 14. This aperture 34 allows insults of fluid or body menses to directly contact the absorbent core 18 which is desirably disposed between the body facing 14 and garment facing layer 16. In this execution, the body facing layer 14 may be partially or entirely hydrophobic.

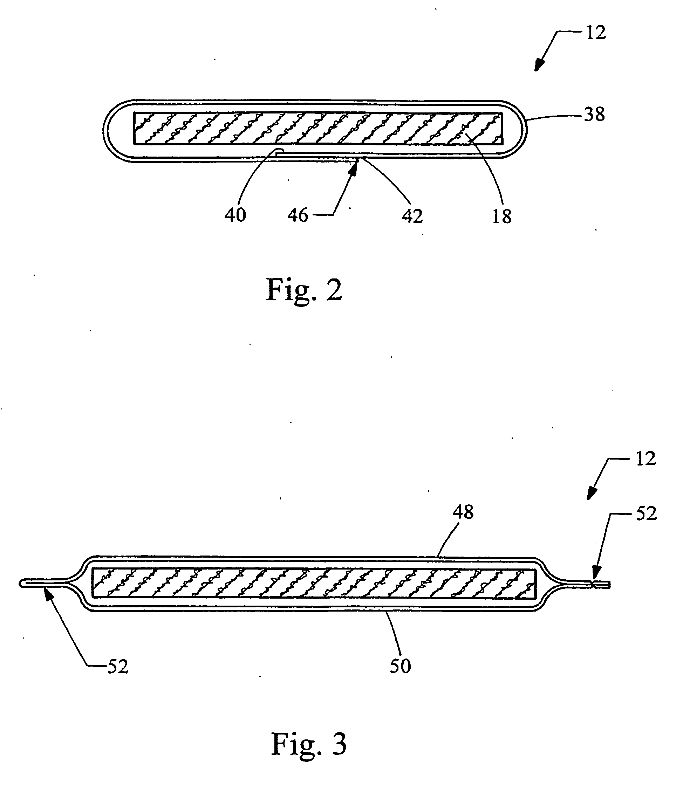

Also, the body facing layer 14 and the garment facing layer 16 may be comprised of one or more individual sheets of extensible material. For example, with reference to FIG. 2, the body facing and garment facing layers may be made of one continuous sheet of extensible material 38. The sheet 38 is wrapped around the absorbent core 18 and opposite ends 40 and 42 of the sheet 38 are affixed to one another at a predetermined location about the article 12. Preferably, the point at which the opposed ends 40 and 42 of the extensible sheet 38 are affixed is located toward the garment facing surface 46 of the absorbent article 12, but may be located at any point on the article 12. If the body facing and garment facing layers are comprised of one individual sheet 38, it is preferable that the material be wrapped about the absorbent core 18 in such a manner as to allow the sheet 38 to extend along the longitudinal axis X. If the core is wrapped in one fluid permeable extensible sheet, as shown, additional fluid impervious material (not shown) may be incorporated to prevent fluid passage to the underwear and outer garments. An example of such a material would be a low gauge polyethylene film. Desirable materials would likewise be extensible. Such materials include biaxial extensible film such as the polyethylene film is described in U.S. Patent No. 6,096,014 to Haffner et al. (see description of garment facing layer, above) and heavy adhesive spray, such as Dispomelt 2525A, which is commercially available from National Starch Corp., Bridgewater, New Jersey, or any other suitable elastomeric adhesive.

In the alternative, the body facing layer and the garment facing layer may be comprised of more than one individual sheet of extensible material, as shown in FIG. 3. As such the absorbent core 18 will desirably be disposed between the sheets 48 and 50 of extensible material that define the body and garment facing layers. The sheets of extensible material 48 and 50 can be coextensive in a face-to-face contact around the outer edge of the absorbent core 18. The sheets 43 and 50 can be sealed together about their peripheries by use of an adhesive, by heat sealing, ultrasonics, embossing, or by any other process known in the art, A suitable hotmelt adhesive, used to seal the peripheries of the absorbent article is Dispomelt 2525Pi and is commercially available from the National Starch Corp. Bridgewater, New Jersey.

Referring again to FIG. 1, this sealed area is the peripheral seal 52. The peripheral seal 52 has end margins 54 and side margins 56. The length and width dimensions of the body facing layer 14 and the garment facing layer 16 are , generally larger than and extend beyond the corresponding dimensions of the absorbent core 18 to provide for the corresponding end margins 54 and side margins 56. The shape, length, and width, of the absorbent article 12 will be defined by the peripheral edges of the body facing layer 14 and garment facing layer 16.

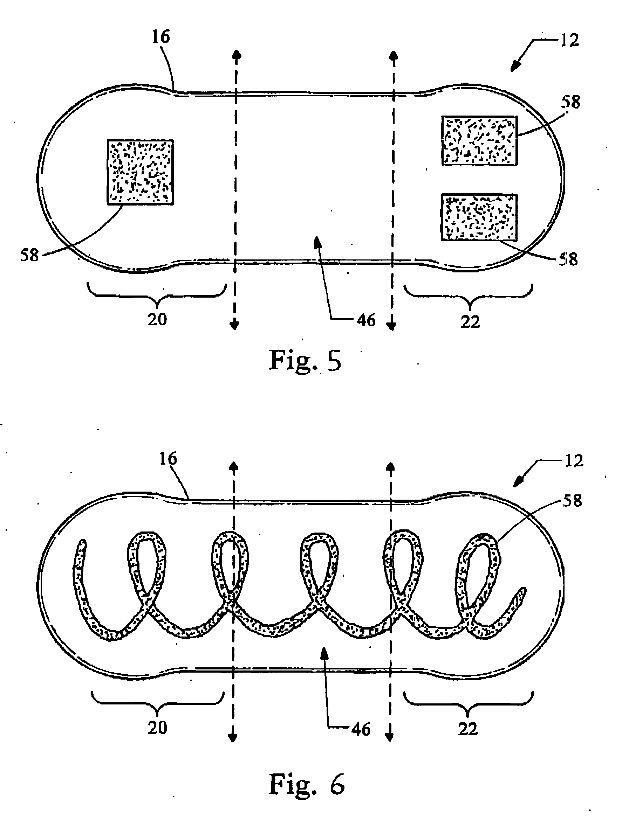

The absorbent article 12 of the present invention may be affixed to the undergarment of the user (not shown) by a variety of methods known to those of skill in the art. Desirably, the absorbent article 12 is extended to the user's desired length by applying pressure to the body facing layer 14 and the garment facing layer 16 along the longitudinal axis X. Once extended, the user may secure the article 12 to the crotch region of her underwear with a releasable attachment component 58 that is desirably disposed on the garment facing layer 16 of the absorbent article 12. The attachment component 58 is desirably disposed in discrete areas about the underside of the garment facing layer 16. More desirably, the attachment component 58 is disposed in discrete areas about the first 20 and second 22 end portions of the garment facing layer 18 as shown in FIG. 5. The placement of the attachment component 58 should not inhibit the extension of the garment facing layer 16,

Likewise, the attachment component 58 may be disposed about the garment facing layer 16 in a discreet or continuous pattern, such as circles, spirals, patches, or dashed lines, allowing the material of the garment facing layer 16 to stretch and extend uninhibited by the attachment component 58, as shown in FIG. 6. The attachment component may be stretchable adhesive or mechanical fasteners. Examples of suitable attachment components are described in U.S. Pat. No. 5,540,673 to Thomas, U.S. Pat. No. 5392498 to Goulalt, and U.S. Pat. No. 6,489,004 to Martin.

Referring again to FIG. 1, the absorbent article 12 of the present invention includes an absorbent core 18. The absorbent core 18 is desirably disposed between the body facing 14 and the garment facing layers 16. The absorbent core 18 will include at least one layer, but as discussed below, the absorbent core 18 may include more than one layer in more than one configuration. Each layer may be comprised of the same or different materials. The absorbent core 18 provides an absorbent structure that is configured for holding and storing absorbed liquids and other waste materials. The absorbent core can be composed of any material that will absorb bodily exudates such as menses, blood, and urine, Suitable absorbents include cellulose fluff pulp, wood fluff, rayon, cotton, superabsorbents, foam, and mixtures thereof. Additionally stabilized absorbents such as airlaid can be used. Meltblown polymers, such as polyester, polypropylene, or combinations thereof, hydroentangled pulp, tissue, and elastic scrim can also be used. Desirably, a thin and flexible absorbent material, such as elastic coform, can be used. Elastic coform can be made according to the process disclosed in U.S. Patent 6,362,389 to McDowall et al., col. 11, lines 66-67 which refers to U.S. Patents 4,818,464 to Lau and 4,104,324 to Anderson et. al . Elastic scrim can be made according to the process disclosed in U.S. 2005/0015068 A1. Superabsorbents on stretchable substrate can be made according to the process disclosed in U.S. 2005/0096623 A1 to Sawyer et al.

As shown in FIG. 1, the absorbent core 18 may include a first absorbent layer 60 and may further include a surge layer 62. Desirably the surge layer 62 is disposed between the body facing layer 14 and the first absorbent layer 60 and may be exposed to the body of the user through the aperture 34 in the body facing layer 14. Suitable materials for use as a surge layer include bonded carded webs including through-air bonded carded webs, bicomponent spunbond, and low basis weight, low density airlaids. Structures composed either entirely or in part of polyester fiber are particularly desirable as a means to impart resiliency and maintain an open, low density structure. Such materials are described in U.S. Patent No. 5.994,615 to Dodge et al. The absorbent core 18 may be affixed to either or both the body facing layer 14 and the garment facing layer 16 at a location between the first and second ends 20 and 22 of the body facing 14 and garment facing 16 layers. The core 18 may be secured to the outer layers 14 and 16 by any suitable method of attachment known In the art.

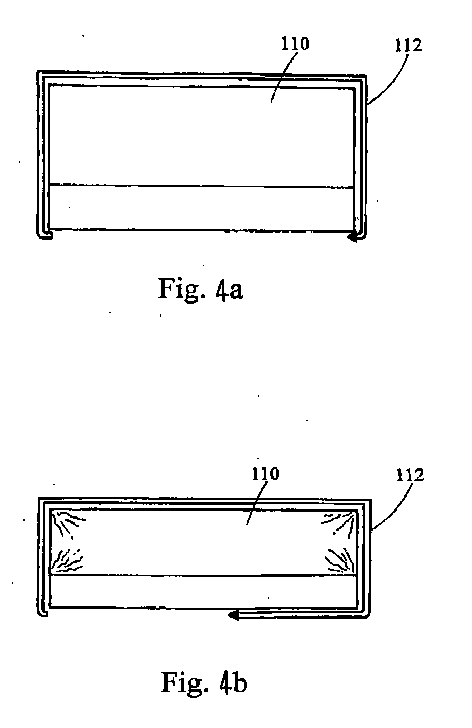

As shown in FIGS. 4 and 4B, another embodiment of the present invention includes an article with extensible body facing layer and/or an extensible garment facing layer, as described above, and an absorbent core 110 that may be tailored to a desired thickness and stabilized to maintain that thickness during use by stretching and refastening an adjustable shell 112 surrounding the absorbent core 110. FIG. 4A shows this embodiment in a pre-use condition. The resilient absorbent core 110 is surrounded by an adjustable shell 112, The user may then adjust the thickness or bulk of the absorbent core 110 by reducing or compressing the circumference of the adjustable shell 112 and stabilizing the absorbent material, FIG. 4B.

Although the present invention has been described with reference to preferred embodiments, those skilled in the art will recognize that changes may be made in form and detail without departing from the scope of the invention. As such, it Is intended that the foregoing detailed description be regarded as illustrative rather than limited and that it is the appended c!aims, including all equivalents thereof, which are intended to define the scope of the invention.

## Claims

1. A customizable absorbent article (12) comprising:
an extensible body facing layer (14) having a first end (20) and a second end (22) spaced along a longitudinal axis, wherein said body facing layer (14) is extensible along said longitudinal axis; and
an extensible garment facing layer (16) having a first end (20) and a second end (22) spaced along said longitudinal axis, wherein said garment facing layer (16) is extensible along said longitudinal axis; **characterised by**
a non-extensible absorbent core (18) disposed between said body facing layer (14) and said garment facing layer (16);
wherein said absorbent core (18) is affixed to at least one of said body facing and said garment facing layer (14, 16) at at least one location between said first and second ends (20, 22); and
wherein said body facing layer (14) has at least one aperture (34) disposed in approximately the center of the body facing layer (14).

2. The customizable absorbent article (12) of claim 1, wherein said at least one aperture (34) exposes a surge layer (62), disposed between said body facing layer (14) and said garment facing layer (16), to a body of a user.

3. The customizable absorbent article (12) of claim 1, wherein said garment-facing layer (16) further comprises a releasable attachment component (58).

4. The customizable absorbent article (12) of claim 3, wherein said attachment component (58) comprises discrete areas of mechanical fastener disposed in a pattern on said garment-facing layer (16).

5. The customizable absorbent article (12) of claim 3, wherein said attachment component (58) comprises discrete areas of mechanical fastener disposed at said first and second ends (20, 22) of said garment-facing layer (16).

6. The customizable absorbent article of claim 1, wherein said absorbent core (18) is anchored to at least said garment facing layer (16) by embossing.

7. The customizable absorbent article (12) of claim 1, wherein said body facing layer (14) and said garment facing layer (16) are comprised of a single piece of extensible material (38) encasing said absorbent core (18).

8. The customizable absorbent article (12) of claim 1, wherein said body facing layer (14) and said garment facing layer (16) are individual sheets of extensible material (48, 50), and wherein said body facing layer (14) and said garment facing layer (16) are attached to one another along outer peripheries of said layers (14, 16).

## Patentansprüche

1. Anpassungsfähiger Absorptionsartikel (12), enthaltend:
eine dehnbare, dem Körper zugewandte Schicht (14), die ein erstes Ende (20) und ein zweites Ende (22) hat, die entlang einer Längsachse beabstandet sind, wobei die dem Körper zugewandte Schicht (14) entlang dieser Längsachse dehnbar ist: und
eine dehnbare, dem Kleidungsstück zugewandte Schicht (16), die ein erstes Ende (20) und ein zweites Ende (22) hat, die entlang einer Längsachse beabstandet sind, wobei die dem Kleidungsstück zugewandte Schicht (16) entlang dieser Längsachse gedehnt werden kann; **gekennzeichnet durch**
einen nicht dehnbaren Absorptionskern (18), der zwischen der dem Körper zugewandten Schicht (14) und der dem Kleidungsstück zugewandten Schicht (16) angeordnet ist;
wobei der Absorptionskern (18) an der dem Körper zugewandten Schicht und/oder der dem Kleiddungsstück zugewandten Schicht (14,16) an wenigstens einer Stelle zwischen dem ersten und dem zweiten Ende (20; 22) befestigt ist; und
wobei die dem Körper zugewandte Schicht (14) wenigstens eine Öffnung (34) aufweist, die etwa in der Mitte der dem Körper zugewandten Schicht (14) angeordnet ist.

2. Anpassungsfähiger Absorptionsartikel (12) nach Anspruch 1, bei dem die wenigstens eine Öffnung (34) eine Abführschicht (62), die zwischen der dem Körper zugewandten Schicht (14) und der dem Kleidungsstück zugewandten Schicht (16) angeordnet ist, für den Körper eines Benutzers freilegt.

3. Anpassungsfähiger Absorptionsartikel (12) nach Anspruch 1, bei dem die dem Kleidungsstück zugewandte Schicht (16) weiterhin ein lösbares Anbringungselement (58) enthält.

4. Anpassungsfählger Absorptionsartikel (12) nach Anspruch 3, bei dem das Anbringungselement (58) diskrete Bereiche einer mechanischen Befestigungseinrichtung enthält, die in einem Muster auf der dem Kleidungsstück zugewandten Schicht (16) angeordnet sind.

5. Anpassungsfähiger Absorptionsartikel (12) nach Anspruch 3, bei dem das Anbringungselement (58) diskrete Bereiche einer mechanischen Befestigungseinrichtung enthält, die am ersten und am zweiten Ende (20, 22) der dem Kleidungsstück zugewandten Schicht (16) angeordnet sind.

6. Anpassungsfähiger Absorptionsartikel (12) nach Anspruch 1, bei dem der Absorptionskern (18) wenigstens an der dem Kleidungsstück zugewandten Schicht (16) durch Prägen verankert ist.

7. Anpassungsfähiger Absorptionsartikel (12) nach Anspruch 1, bei dem die dem Körper zugewandte Schicht (14) und die dem Kleidungsstück zugewandte Schicht (16) aus einem einzigen Stück eines dehnbaren Materials bestehen, das den Absorptionskem (18) einschließt.

8. Anpassungsfähiger Absorptionsartikel (12) nach Anspruch 1, bei dem die dem Körper zugewandte Schicht (14) und die dem Kleidungsstück zugewandte Schicht (16) einzelne Bögen eines dehnbaren Materials (48, 50) sind und bei dem die dem Körper zugewandte Schicht (14) und die dem Kleidungsstück zugewandte Schicht (16) entlang der Außenränder dieser Schichten (14, 16) miteinander verbunden sind.

## Revendications

1. Article absorbant personnalisable (12) comportant:
une couche extensible orientée vers le corps (14) présentant une première extrémité (20) et une deuxième extrémité (22) séparées le long d'un axe longitudinal, ladite couche orientée vers le corps (14) étant extensible le long dudit axe longitudinal ; et
une couche extensible orientée vers le vêtement (16) présentant une première extrémité (20) et une deuxième extrémité (22) espacées le long dudit axe longitudinal, ladite couche orientée vers le vêtement (16) étant extensible le long dudit axe longitudinal ;
**caractérisé par**
un noyau absorbant non-extensible (18) disposé entre ladite couche orientée vers le corps (14) et ladite couche orientée vers le vêtement (16) ;
ledit noyau absorbant (18) étant fixé sur au moins une desdites couche orientée vers le corps et couche orientée vers le vêtement (14, 16) au niveau d'au moins un emplacement entre lesdites première et deuxième extrémités (20, 22) ; et
ladite couche orientée vers le corps (14) ayant au moins une ouverture (34) disposée à peu près au centre de la couche orientée vers le corps (14).

2. Article absorbant personnalisable (12) selon la revendication 1, dans lequel ladite au moins une ouverture (34) expose une couche épongeante (62), disposée entre ladite couche orientée vers le corps (14) et ladite couche orientée vers le vêtement (16), au corps d'un utilisateur.

3. Article absorbant personnalisable (12) selon la revendication 1, dans lequel ladite couche orientée vers le vêtement (16) comporte en outre un élément d'attache amovible (58).

4. Article absorbant personnalisable (12) selon la revendication 3, dans lequel ledit élément d'attache (58) comporte des zones discrètes de fixations mécaniques disposées selon un motif sur ladite couche orientée vers le vêtement (16).

5. Article absorbant personnalisable (12) selon la revendication 3, dans lequel ledit élément d'attache (58) comporte des zones discrètes de fixations mécaniques disposées au niveau desdites première et deuxième extrémités (20, 22) de ladite couche orientée vers le vêtement (16).

6. Article absorbant personnalisable selon la revendication 1, dans lequel ledit noyau absorbant (18) est ancré à au moins ladite couche orientée vers le vêtement (16) par un gaufrage.

7. Article absorbant personnalisable (12) selon la revendication 1, dans lequel ladite couche orientée vers le corps (14) et ladite couche orientée vers le vêtement (16) comprennent une seule pièce de matériau extensible (38) enveloppant ledit noyau absorbant (18).

8. Article absorbant personnalisable (12) selon la revendication 1, dans lequel ladite couche orientée vers le corps (14) et ladite couche orientée vers le vêtement (16) sont des feuilles individuelles de matériau extensible (48, 50), et dans lequel ladite couche orientée vers le corps (14) et ladite couche orientée vers le vêtement (16) sont attachées l'une à l'autre le long des bords externes desdites couches (14, 16).
